# EUROPÄISCHE PATENTANMELDUNG

(11) **EP 0 867 159 A1**
(43) Veröffentlichungstag der Anmeldung: **30.09.1998**
(21) Anmeldenummer: 97810176.4
(22) Anmeldetag: 25.03.1997
(51) Int. Cl.: A61F 2/34, B21D 24/00, B21J 15/00, B05C 17/005

(54) **Zweischalige künstliche Hüftgelenkpfanne und deren Herstellung**

(71) Anmelder: Sulzer Orthopädie AG, 6340 Baar (CH)
(72) Erfinder: Gross, Walter, 8475 Ossingen (CH); Bider, Kurt, 8406 Winterthur (CH); Oehy, Jürg, 8444 Henggart (CH)
(74) Vertreter: Heubeck, Bernhard

(57) **Zusammenfassung**

Mit der Erfindung wird eine Hüftgelenkschale und deren Herstellung gezeigt, wobei die Hüftgelenkschale aus einer dünnwandigen metallischen Aussenschale (1) mit Verankerungszapfen (8) besteht, die fest mit einer Innenschale (2) aus Kunststoff verbunden ist. Da die Lagerfläche (21) der Innenschale (2) erst nach dem Verbund erzeugt wird, ergibt sich eine hohe Formgenauigkeit der Lagerfläche zusammen mit einer relativ elastischen Aussenschale und mit guten Verankerungshilfen für eine Primärverankerung.

## Beschreibung

Die Erfindung handelt von einer zweischaligen künstlichen Hüftgelenkpfanne mit einer Aussenschale aus Titan und einer Innenschale aus hochmolekular verdichteten Polyethylen, wobei die Aussenschale die Innenschale im Bereich des Äquators soweit nach innen überragt, dass eine dauerhafte, nicht mehr lösbare Verbindung zwischen den Schalen besteht.

Eine Hüftgelenkschale bei der die Aussenschale aus Metall und die Innenschale aus Polyethylen besteht, ist in der US-A-5,080,677 gezeigt. Die Aussenschale wird in einem dafür vorbereiteten Beckenknochen verankert, wobei auch Knochenschrauben zu Hilfe genommen werden können, die durch Oeffnungen in der Aussenschale in den Beckenknochen eingezogen werden können. Die Innenschale weist bei Raumtemperatur gegenüber der Aussenschale ein Uebermass auf, das sich durch Unterkühlen der Innenschale und entsprechendes Schrumpfen der Innenschale soweit zurückbildet, dass die unterkühlte Innenschale intraoperativ eingesetzt werden kann und mit dem Erreichen einer Körpertemperatur von 37°C durch ihre Ausdehnung einen Pressitz erzeugt, der den Kunststoff zusätzlich zum Fliessen in Hinterschneidungen an der Aussenschale bringt, um so eine dauerhafte Verbindung zu erzeugen.

Im weiteren offenbart die EP-A-0 699 425 unter anderem eine Hüftgelenkschale mit einer Aussenschale aus Titan, die an ihrem Äquator um eine Innenschale aus Polyethylen mit einwärts stehender Gegenfläche eingerollt ist. Es gibt weiterhin Zapfen, die mit einer Klemmverbindung radial in Aussenschalen verankert sind (US-A-5,226,917). Dies setzt aber eine dickwandige Aussenschale mit all ihren Fertigungsnachteilen voraus.

Aufgabe der vorliegenden Erfindung ist es eine als Massenfabrikation kostengünstige Prothese zu schaffen, die gut verankerbar ist.

Diese Aufgabe wird mit den Kennzeichen vom Anspruch 1 dadurch gelöst, dass dass die Aussenschale aus einem Tiefziehblech mit einer Dicke 3 > s > 1 mm geformt ist, dass die Aussenschale durch Bördeln oder Einrollen um eine Schulter der Innenschale eingerollt ist, und dass die Aussenschale annähernd in einer Hauptbelastungsrichtung mindestens eine Ausstülpung mit einem daran befestigten Verankerungszapfen aufweist.

Ein Vorteil der Erfindung liegt in einem breiten Anwendungsbereich, da sie in relativ vielen Fällen anwendbar ist. Ein weiterer Vorteil liegt darin, dass aufgehärtete Polyethylensorten, die durch Bestrahlung mit Elektronen- oder γ-Strahlen und eine Wärmebehandlung einen höheren Vernetzungsgrad und weniger Radikale enthalten, problemlos für Innenschalen mit der gezeigten Herstellung eingesetzt werden können, da weder die Formgenauigkeit der Lagerfläche beeinträchtigt wird, noch ein Fliessen vom Polyethylen in die Aussenschale vorausgesetzt wird. Mit einem Polyethylen, das eine deutlich geringere Verschleissrate aufweist, entfällt für viele Fälle auch die Notwendigkeit eine Innenschale austauschbar zu machen. Dies hat nicht nur zur Folge, dass Reoperationen aus Verschleissgründen entfallen, sondern dass die Gestaltung vom Verbund auf besseres Funktionsverhalten hin optimiert werden kann. Dabei hat sich gezeigt, dass eine dünne Aussenschale aus Titan einen relativ geringen Widerstand zwischen einer elastischen Innenschale aus Polyethylen und einem elastischen Knochenbett darstellt. Die Aussenschale sollte also den sich ergebenden Deformationen zwischen Innenschale und Knochenbett folgen können und trotzdem primär und sekundär gut verankerbar sein. Als eine Art Optimum oder bester Kompromiss hat sich eine Aussenschale ergeben, die aus einem Tiefziehblech aus Titan mit einer Dicke 3 > s > 1 mm hergestellt wird und nach dem nachstehend beschriebenen Verfahren bearbeitet wird, wobei eine Blechdicke von 2 mm bevorzugt wird. Eine Rondelle aus Tiefziehblech wird mit einem Stempel in eine Matrize gepresst und erhält die Form einer Schale mit zylindrischem Fortsatz. Nach dem Ausstossen des Werkstückes wird der Einlaufkragen im zylindrischen Teil von der Aussenschale beispielsweise durch Drehen abgetrennt, wobei ein zylindrischer Rand an der Schale stehen bleibt. Zur besseren Zentrierung der Aussenschale bei den Folgeoperationen kann eine Zentrierbohrung am Pol der Aussenschale angebracht werden.

Mit weiteren Presswerkzeugen werden eine oder mehrere Ausstülpungen an der Aussenschale ausgeformt und Innenschultern für die spätere Befestigung der Verankerungszapfen gebildet. Es können weitere Vorsprünge in Form von Zacken ausgeformt werden, die bei der Primärverankerung der Aussenschale in einem Knochenbett eine Hilfe sind.

Vor dem Auspressen der endgültigen Form der Ausstülpungen kann es von Vorteil sein, durch ein Zwischenglühen die Vorspannungen herabzusetzen. In den Ausstülpungen werden Bohrungen angebracht, in welche die Verankerungszapfen von aussen aufgesteckt werden, um sie mit den Innenschultern der Ausstülpungen zu vernieten.

Durch die Verwendung eines hohlen, taumelnden Nietwerkzeuges können die Verankerungszapfen so ausgebildet werden, dass auf der Innenseite eine Positionierspitze für die spätere Innenschale stehen bleibt.

Durch die Verwendung eines dünnen Tiefziehbleches für die Aussenschale ist es auch möglich, vorverformte Metallgewebe "mesh" zum Beispiel im Bereich der Verankerungszapfen auf der Aussenschale aufzupunkten, um ein Einwachsen von Knochengewebe zu begünstigen.

Für die spätere Innenschale wird ein Rundling aus verschleissfestem Kunststoff beispielsweise aus aufgehärtetem hochmolekularen Polyethylen mit einer Aussenkontur versehen, die der Innenkontur der Aussenschale bis auf einen Rücksprung im zylindrischen Äquatorbereich entspricht. Der Kunststoffrundling wird nun in einem Werkzeug in die Aussenschale gepresst und gleichzeitig wird der zylindrische Rand der Aussenschale durch Einrollen oder Bördeln fest mit der späteren Innenschale verbunden. Durch ein Vorwärmen der Aussenschale weit über Sterilisationstemperatur kann das Material vom Rundling beim Pressen auch in Hohlräume fliessen, die beim Ausformen von Vorsprüngen an der Aussenschale entstanden sind. Dies hat den Vorteil, dass keine Hohlräume stehen bleiben, die die Ansiedlung von Keimen fördern.

Der mit der Aussenschale fest verbundene Rundling wird auf der Innenseite durch spanende Formgebung zu einer Lagerschale fertig bearbeitet. Zusätzlich können Bohrungen für Knochenschrauben an der Stirnfläche der Innenschale angebracht werden, wobei dazu die Aussenschale mit einem passenden Fräser von aussen geöffnet wird.

Die hier beschriebenen Hüftgelenkschalen und ihre Fertigung haben den Vorteil, dass sie für eine kostengünstige Herstellung bei grossen Stückzahlen geeignet sind. Ein weiterer Vorteil ist, dass die Verbindung von Verankerungszapfen zu einer sehr dünnen Aussenschale unter kontrollierten Werkstattbedingungen, d.h. ohne Einfluss des Chirurgen stattfindet. Weiterhin ist es von Vorteil, dass die Aussenschale so dünn gehalten werden kann, dass sie den Deformationen von Knochenbett und Innenschale besser folgen kann.

Im folgenden wird die Erfindung näher beschrieben. Es zeigen:
- Figur 1: Schematisch die untere Ansicht einer erfindungsgemässen Hüftgelenkschale;
- Figur 2: schematisch die Seitenansicht der Hüftgelenkschale von Figur 1;
- Figur 3: schematisch die Draufsicht der Hüftgelenkschale von Figur 1;
- Figur 4: schematisch einen vergrösserten Schnitt durch die Hüftgelenkschale von Figur 3;
- Figur 5: schematisch einen vergrösserten Schnitt durch die Hüftgelenkschale von Figur 2 mit zwei Verankerungszapfen;
- Figur 6: schematisch einen zu Figur 4 versetzten Ausschnitt, mit ausgepressten Vorsprüngen in Form von Zacken,
- Figur 7: schematisch einen vergrösserten Ausschnitt eines in eine Ausstülpung eingesetzten Verankerungszapfens vor dem Vernieten;
- Figur 8: schematisch einen Schnitt durch ein Tiefziehwerkzeug für die Herstellung einer dünnwandigen Aussenschale;
- Figur 9: schematisch einen Schnitt durch ein Tiefziehwerkzeug für die Herstellung von Ausstülpungen an einer metallischen Aussenschale;
- Figur 10: schematisch einen Schnitt durch ein Presswerkzeug zum Ausformen von zwei parallelen Ausstülpungen;
- Figur 11: schematisch einen Querschnitt durch den Werkzeugoberteil und eine Draufsicht auf den Werkzeugunterteil vom Presswerkzeug Figur 10;
- Figur 12: schematisch ein Press- und Einrollwerkzeug zum Verbinden einer tiefgezogenen Aussenschale mit einer späteren Innenschale und
- Figur 13: schematisch ein Nietwerkzeug und eine Haltevorrichtung für das Vernieten von Verankerungszapfen mit einer Ausstülpung einer dünnwandigen Aussenschale.

Mit den Figuren wird eine Hüftgelenkschale und deren Herstellung gezeigt, wobei die Hüftgelenkschale aus einer dünnwandigen metallischen Aussenschale 1 mit Verankerungszapfen 8 besteht, die fest mit einer Innenschale 2 aus Kunststoff verbunden ist. Da die Lagerfläche 21 der Innenschale 2 erst nach dem Verbund erzeugt wird, ergibt sich eine hohe Formgenauigkeit der Lagerfläche zusammen mit einer relativ elastischen Aussenschale und mit guten Verankerungshilfen für eine Primärverankerung.

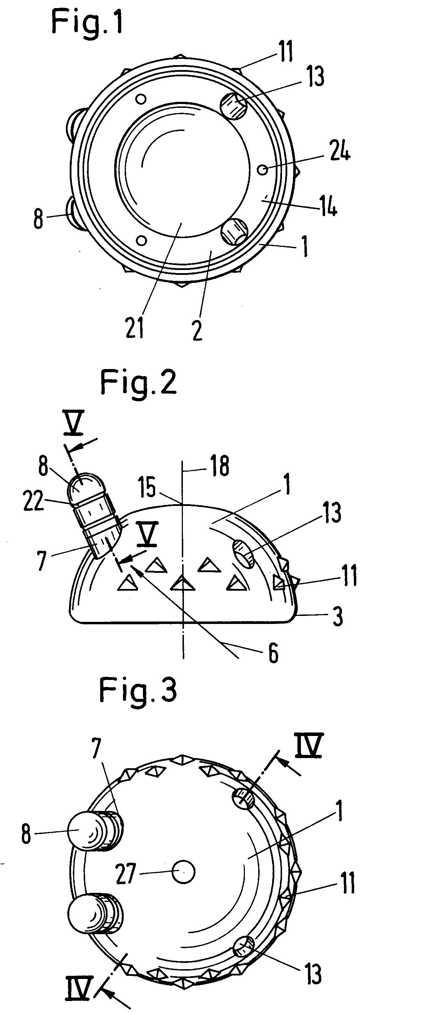

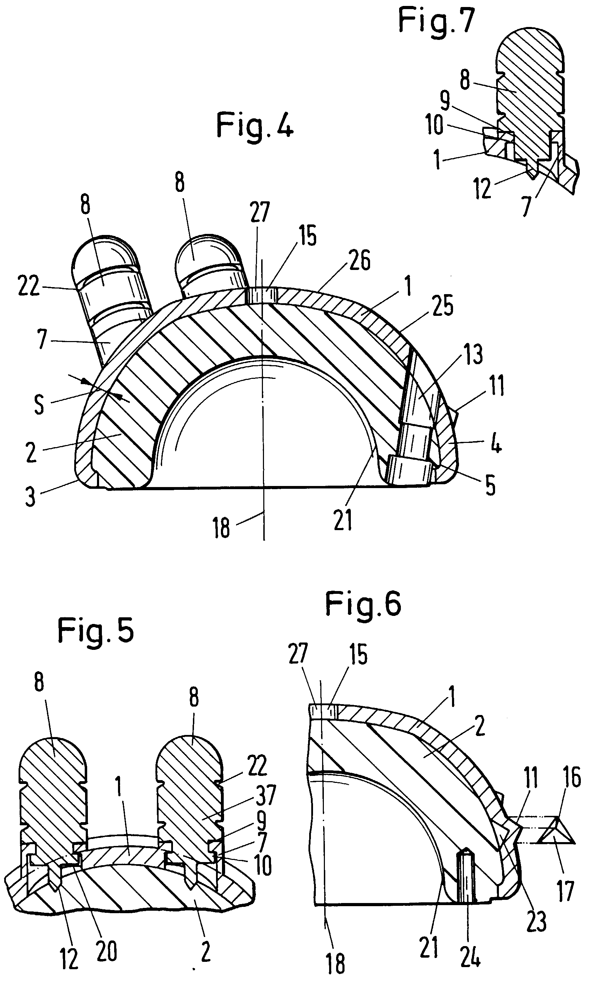

Die Figuren 1, 2, 3 und 4 zeigen eine Hüftgelenkschale mit einer Aussenschale 1 und einer darin eingerollten Innenschale 2. Die Aussenschale 1 besteht aus einem Tiefziehblech 4 aus Titan mit einer Dicke S von 2 mm. Aus dem Dach der Aussenschale 1 sind zwei Ausstülpungen 7 ausgepresst, die jeweils einen Sockel für einen fest verbundenen Verankerungszapfen 8 bilden. Die Verankerungszapfen 8 sind parallel zueinander angeordnet und sind Drehteile aus Titan, die Einstiche 22 zur besseren Verankerung im Knochen aufweisen. Die Ausstülpungen 7 sind im Bereich einer Hauptbelastungskraft 6 vorgesehen. Die Neigung der Verankerungszapfen 8 zur Polachse 18 ist so gewählt, dass die Schale 1 der Richtung der Zapfen 8 beim Einsetzen in ein Knochenbett folgen kann. Oberhalb des Äquators 3 der Aussenschale sind Zacken 11 herausgedrückt, die der besseren Primärverankerung dienen. Von der Stirnseite 14 der Innenschale 2 gehen Schrägbohrungen 13 durch die Aussenschale 1 hindurch, um mit darin einsetzbaren Knochenschrauben die Verankerung zu unterstützen. Am Pol 15 der Aussenschale 1 ist eine Zentrier- und Kontrollöffnung 27 angebracht, um die Aussenschale zentrieren zu können und um den Verbund von Aussen- und Innenschale kontrollieren zu können. Auf der Stirnseite 14 der Innenschale 2 sind drei Aufnahmebohrungen 24 angebracht, um die fertige Schale 1, 2 mit einem Setzwerkzeug aufnehmen zu können. Die Innenschale 2 besitzt eine kugelförmige Lagerfläche 21, während ihre Aussenfläche wie auch bei der Aussenschale 1 eine Kugelfläche 25 mit Abplattung 26 im Polbereich 15 aufweist. In Figur 4 ist im Bereich vom Äquator 3 eine Schulter 5 erkennbar, um die die Aussenschale 1 eingerollt ist.

In Figuren 7 und 5 ist die Lage von Verankerungszapfen 8 vor und nach dem Vernieten mit einer entsprechenden Ausstülpung 7 der Aussenschale 1 zu sehen. Der Verankerungszapfen 8 wird an einer Bohrung 9 der Ausstülpung 7 aufgesteckt und steht über die Innenschulter 10 in die Aussenschale hinein, wobei eine Positionierspitze 12 für die spätere Innenschale 2 am weitesten vorsteht. Beim eigentlichen Nietvorgang wird das Material um die Positionierspitze herum zur Innenschulter 10 hin plastisch verformt, um mit dieser einen festen Verbund durch einen Nietkopf 20 zu bilden. Der Verankerungszapfen 8 muss von aussen aufgesteckt werden, weil er mit seinem Kopf 37 über die Bohrung 9 bis zum Aussendurchmesser der Ausstülpung 7 vorsteht.

In Figur 6 ist zusätzlich ein Schnitt durch eine Zacke 11 gezeigt, die aus der Aussenschale herausgedrückt ist und die die Form einer Dreieckpyramide hat, wobei eine Kante als Schneide 16 zum Pol 15 hin orientiert ist und eine Flachseite 17 zum Äquator hin ausgerichtet ist. Vom Herausdrücken der Zacke 11 ist auf der Innenseite der Aussenschale 1 eine Hohlstelle 23 stehen geblieben.

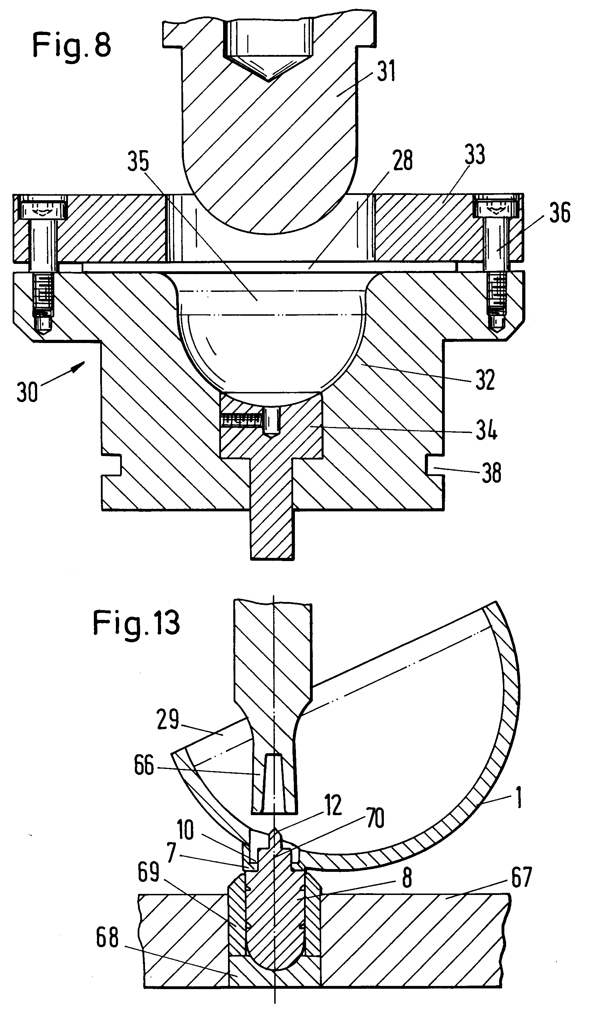

Mit den Figuren 8 bis 13 werden verschiedene Werkzeuge für die Herstellung der Hüftgelenkschale 1, 2 beschrieben.

In Figur 8 ist ein Tiefziehblech 28 in einem Tiefziehwerkzeug 30 eingelegt. Das Werkzeug besteht aus einem Stempel 31 und einer Matrize 32, welche an ihrem Boden durch einen Ausstosser 34 ergänzt wird. Ein Niederhalter 33 ist über Halteschrauben 36 an der Matrize 32 befestigt. Die Innenseite der Matrize 32 besitzt einen sphärischen Bereich, an den sich ein zylindrischer Bereich 35 anschliesst. Nach dem Tiefziehen wird die Aussenschale vom Rohling in diesem zylindrischen Bereich 35 abgestochen, wobei ein zylindrischer Kragen 29 für das spätere Einrollen an der Aussenschale stehen bleibt.

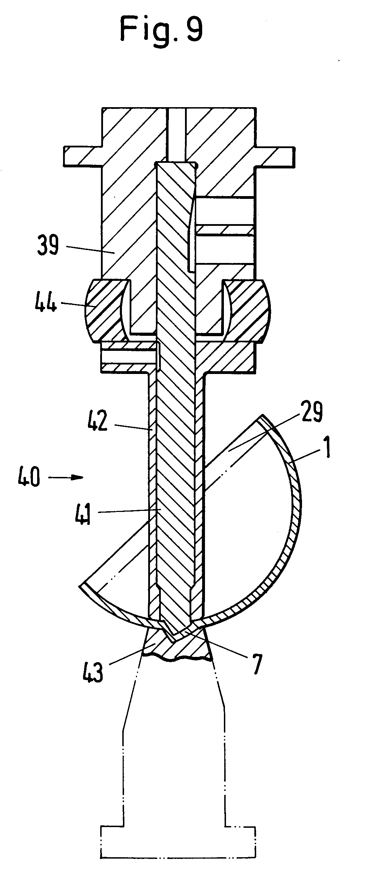

In Figur 9 ist ein Tiefziehwerkzeug 40 gezeigt mit einer Matrize 43, die die Form der Ausstülpung 7 aufweist, und mit einem Werkzeugoberteil 39. Am Werkzeugoberteil wird ein Niederhalter 42 über ein Federelement 44 gegen eine eingelegte Aussenschale 1 vorgespannt, bevor ein Stempel 41 das Blech in die Matrize 7 hineindrückt und so eine Ausstülpung 7 in ihrer Rohform erzeugt. Da zwei Ausstülpungen 7 parallel zueinander angeordnet sind, aber keine gemeinsame Ebene durch die Polachse aufweisen, braucht jede der beiden Ausstülpungen 7 ein eigenes Werkzeug.

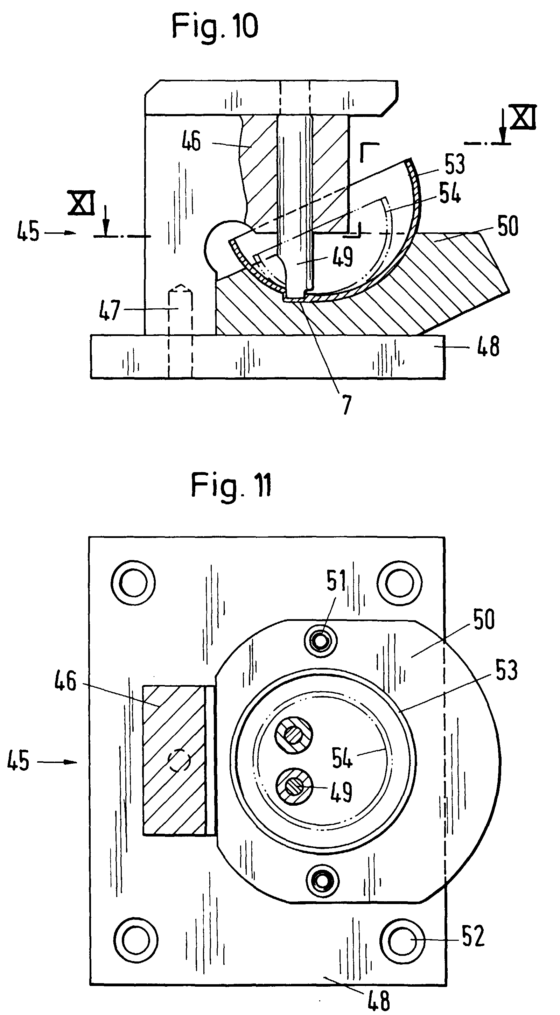

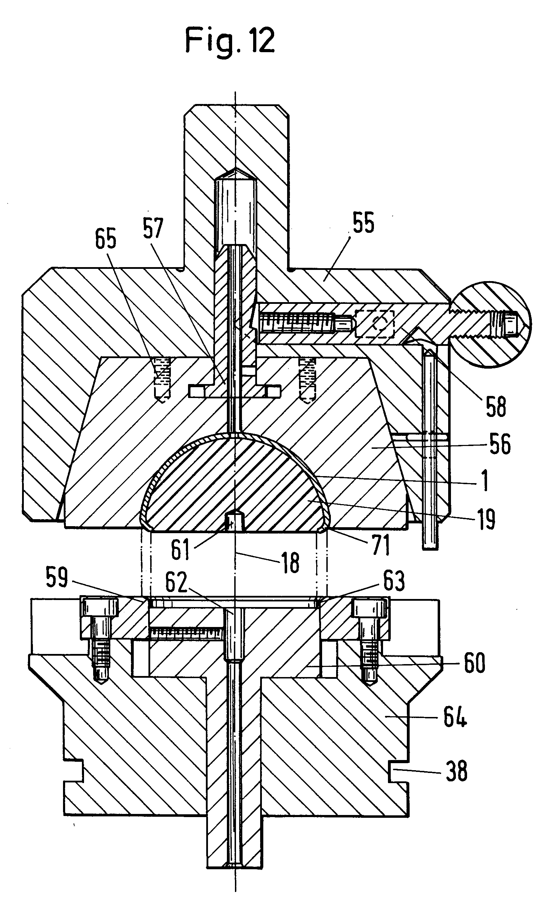

In Figuren 10 und 11 ist ein Auspresswerkzeug 45 für die endgültige Formgebung von zwei Ausstülpungen 7 gezeigt. Eine grosse Aussenschale 53 ist in einer Matrize 50 eingelegt, die ihrerseits über Verschraubungen 51 mit einem Werkzeugunterteil 48 verbunden ist. Ein Werkzeugoberteil 46 wird über einen Stift 46 und die Matrize 50 vorzentriert. Im Werkzeugoberteil 46 sind zwei Stempel 49 integriert, die die endgültige Form der Ausstülpungen 7 masshaltig auspressen. Für eine kleinere Aussenschale 54 wird die Matrize 50 ausgewechselt, während die Stempel 49 stehen bleiben können. Vor dem endgültigen Auspressen der Ausstülpungen 7 kann es notwendig sein, die Aussenschale einem Zwischenglühen zu unterwerfen. Die Presswerkzeuge 30, 40, 45 werden mit Befestigungsschrauben 52 oder Aufnahmenuten 38 in einer Presse beispielsweise in einer 4-Säulenpresse eingespannt. Die Bohrungen 9 in den Ausstülpungen 7 sowie die Zentrierbohrung 27 am Pol 15 werden am einfachsten mit einer Bohrlehre gebohrt.

In Figur 13 ist das Vernieten der Verankerungszapfen 8 mit der Aussenschale 1 gezeigt. In einer Aufnahmevorrichtung 67, die ein Druckteil 68 und eine Führungsbüchse 69 aufweist, wird ein Verankerungszapfen 8 eingelegt und die Aussenschale 1 mit der Ausstülpung 7 aufgesteckt und niedergehalten. Ein Nietwerkzeug 66, das ohne um die eigene Achse zu drehen, eine Taumelbewegung um die eigene Achse macht, wird in Richtung der Zapfenachse 70 zugestellt. Die Innenseite des Nietwerkzeuges 66 besitzt eine Ausnehmung, damit die Positionierspitze 12 des Verankerungszapfens trotz der Taumelbewegung unbeschädigt bleibt. Mit dem taumelnden Werkzeug 66 wird die Schulter ausserhalb der Positionierspitze 12 soweit hinuntergehämmert, dass sich auf der Innenschulter 10 ein Nietkopf 20 bildet. Nach dem Nietvorgang kann eine Rissprüfung an der Aussenschale vorgenommen werden und können Oberflächen kugelgestrahlt und gereinigt werden. Ebenso können die Ausstülpungen 7 vor dem Nieten feingestrahlt und gereinigt werden.

Im Werkzeug von Figur 12 ist das eigentliche Einrollen der Aussenschale 1 um einen Rundling 19 vorgesehen, dessen Aussenkontur der Aussenschale bis auf den zylindrischen Kragen 29 entspricht, welcher vor dem Einrollen über eine Schulter 5 am Äquator 3 vorsteht. Der Rundling 19 besteht aus Vollmaterial, einem verschleissfesten Kunststoff wie beispielsweise aufgehärtetem hochmolekulraren Polyethylen. Das Werkzeugoberteil 55 besitzt einen 4-fach geteilten Formteil 56 dessen Einsätze längs Schrägflächen verschieblich sind, wobei die Einsätze durch eine Führungseinrichtung 57 auf gleicher Höhe gehalten werden und beim Zusammenfahren des Werkzeuges zur Mitte hin bewegt werden. Halteschrauben 65 verhindern ein Herausfallen der Einsätze. Die Führungseinrichtung 57 ist ihrerseits in der geschlossenen Stellung über einen Verriegelungsmechanismus 58 verriegelbar und entriegelbar. Im Werkzeugunterteil 64 ist ein Stempel 60 gelagert, der aussen durch einen Formring 59 begrenzt ist. Zum Einlegen von Rundling 19 und Aussenschale 1 steht der Stempel 60 über den Formring 59 vor, das Werkzeugoberteil ist abgehoben und die Einsätze 56 hängen in ihrer tiefsten Stellung und liegen an den Schrägflächen an, weil sie durch hier nicht gezeigte Federn auseinandergetrieben werden. In eine Zentrierbohrung 62 im Stempel 60 wird ein Zapfen eingesteckt, an dem der Rundling 19 mit seiner Zentrierbohrung 61 beim Aufstecken so zentriert wird, dass an seiner Peripherie ein Hohlraum 63 entsteht, in den der Kragen 29 beim späteren Einrollen hineindeformieren kann. Die Aussenschale 1 mit zylindrischem Kragen 29 wird auf den Rundling 19 am vorstehenden Stempel 60 aufgesteckt. Beim Zusammenfahren des Werkzeuges werden zunächst die Einsätze 56 im Werkzeugoberteil über den vorstehenden Stempel 60, den Rundling 19 und die Aussenschale 1 angehoben und bewegen sich entsprechend den Schrägflächen zur Mitte hin. Gleichzeitig entsteht eine innige Verpressung von Rundling und Aussenschale, wobei ein Einrollen vom Kragen 29 einsetzt, welches mit dem Zurückweichen des Stempels 60 durch den unteren Formring 59 unterstützt wird. Der Kragen legt sich so unter Vorspannung um die Schulter 5 der späteren Innenschale 2. Mit dem Oeffnen des Werkzeugs und Entriegelung der Führungseinrichtung wird das Werkstück 1, 19 freigegeben und es verbleibt nur noch eine spanende Fertigbearbeitung vom Rundling 19 zur Innenschale 2 mit Lagerfläche 21. Da die Aussenschale beim Aufstecken auf den Rundling 19 ausser dem eher isolierenden Kunststoff vom Rundling keine Metallteile berührt und von wärmeisolierender Luft umgeben ist, kann die Aussenschale in vorgewärmtem Zustand beispielsweise mit einer Temperatur von 200°C aufgesteckt werden und das Werkzeug schnell zusammengefahren werden, um den Kunststoff zum Einfliessen in Hohlräume 23 der Aussenschale zu bringen.

Die hier beschriebenen Werkzeuge und die mit ihnen verknüpften Teilschritte können auch als unabhängige Fertigungsschritte bei anderen Implantaten mit dünnwandigen Blechen eingesetzt werden. Ihre Anwendung bezieht sich dann auf ein Implantat mit einem dünnwandigen Blech.

## Patentansprüche

1. Zweischalige künstliche Hüftgelenkschale mit einer Aussenschale (1) aus Titan und einer Innenschale (2) aus hochmolekular verdichteten Polyethylen, wobei die Aussenschale (1) die Innenschale (2) im Bereich des Äquators (3) soweit nach innen überragt, dass eine dauerhafte, nicht mehr lösbare Verbindung zwischen den Schalen besteht, dadurch gekennzeichnet, dass die Aussenschale aus einem Tiefziehblech (4) mit einer Dicke 3 > s > 1 mm geformt ist, dass die Aussenschale (1) durch Bördeln oder Einrollen um eine Schulter (5) der Innenschale (2) eingerollt ist, und dass die Aussenschale (1) annähernd in einer Hauptbelastungsrichtung (6) mindestens eine Ausstülpung (7) mit einem daran befestigten Verankerungszapfen (8) aufweist.

2. Künstliche Hüftgelenkschale nach Anspruch 1, dadurch gekennzeichnet, dass das Tiefziehblech (4) eine Dicke s = 2 mm aufweist.

3. Künstliche Hüftgelenkschale nach Anspruch 1 oder 2, dadurch gekennzeichnet, dass zwei Verankerungszapfen (8) parallel zueinander angeordnet sind.

4. Künstliche Hüftgelenkschale nach einem der Ansprüche 1 bis 3, dadurch gekennzeichnet, dass die Ausstülpung (7) eine Bohrung (9) mit Innenschulter (10) aufweist, an denen der von aussen durchgesteckte Zapfen (8) durch Vernieten von innen befestigt ist.

5. Künstliche Hüftgelenkschale nach einem der Ansprüche 1 bis 4, dadurch gekennzeichnet, dass der Zapfen (8) eine bis in die Innenschale eindringende Positionierspitze (12) als Verdrehsicherung aufweist.

6. Künstliche Hüftgelenkschale nach einem der Ansprüche 1 bis 5, dadurch gekennzeichnet, dass an der Aussenschale durch plastisches Verformen dreieckige Zacken (11) herausgedrückt sind, die eine Schneide (16) zum Pol (15) und eine Flachseite (17) zum Äquator (3) der Aussenschale aufweisen.

7. Künstliche Hüftgelenkschale nach einem der Ansprüche 1 bis 5, dadurch gekennzeichnet, dass sie mindestens im Bereich des oder der Zapfen (8) eine Kappe in Form von einem Netz aus Titandraht aufweist.

8. Verfahren zum Herstellen einer Hüftgelenkschale nach einem der Ansprüche 1 bis 7, dadurch gekennzeichnet, dass
- in einem ersten Schritt eine der Innenschale bis zum höchsten Punkt am Äquator entsprechende und eine daran anschliessende zylindrische Innenkontur an einem Tiefziehblech (28) von einer Dicke 3 > s < 1 mm, vorzugsweise jedoch 2 mm, erzeugt wird,
- in einem der weiteren Schritte die so entstandene Aussenschale (1) im zylindrischen Bereich (35) abgeschnitten wird,
- in einem zweiten Schritt ein oder mehr Ausstülpungen (7) an der Aussenschale mit Presswerkzeugen (30, 45) geformt und mit Schultern (10) versehen werden,
- in einem weiteren Schritt Bohrungen (9) an den Ausstülpungen (7) angebracht werden,
- in einem dritten Schritt von aussen Verankerungszapfen (8) mit überstehendem Kopf (37) in die Bohrungen (9) eingesetzt und von innen vernietet werden,
- in einem vierten Schritt ein Rundling (19) aus Kunststoff beispielsweise aus hochmolekular verdichtem Polyethylen, dessen äussere Kontur der Kontur der Innenschale (2) entspricht, in die Aussenschale (1) gepresst wird, während gleichzeitig durch ein Bördeln oder Einrollen der Rand der Aussenschale zu ihrer Polachse (18) hin verformt wird, um eine nicht lösbare, feste Verbindung zu erzeugen,
- in einem fünften Schritt das Werkstück aus Aussenschale (1) und Rundling (19) an der Aussenschale aufgenommen wird, um spanend den Rundling (19) zur Innenschale mit Lagerfläche (21) fertig zu bearbeiten.

9. Verfahren nach Anspruch 8, dadurch gekennzeichnet, dass in einem Zwischenschritt spätestens vor dem vierten Schritt mit einem Presswerkzeug dreieckige Zacken (11) aus der Aussenschale (1) herausgedrückt werden.

10. Verfahren nach Anspruch 8, dadurch gekennzeichnet, dass in einem Zwischenschritt, spätestens jedoch vor dem vierten Schritt, ein Netz aus Titandraht, welches der Form der Aussenschale (1) entspricht und diese in einem bestimmten Bereich überdeckt, an der Aussenschale durch Schweissen befestigt wird.
